# EUROPEAN PATENT APPLICATION

(11) **EP 4 778 475 A1**
(43) Date of publication of application: **22.07.2026**
(21) Application number: 24938359.7
(22) Date of filing: 04.06.2024
(51) Int. Cl.: A61B 17/50, A61B 1/273

(54) **DIGESTIVE TRACT STENT RETRIEVAL APPARATUS AND SLEEVE FOR ENDOSCOPE**

(30) Priority: 17.05.2024 CN 202410618308
(71) Applicant: Hangzhou Tangji Medical Technology Co., Ltd., Hangzhou, Zhejiang 310052 (CN)
(72) Inventor: LI, Wenyu, Hangzhou, Zhejiang 310052 (CN); HUANG, Peng, Hangzhou, Zhejiang 310052 (CN); LV, Ke, Hangzhou, Zhejiang 310052 (CN); ZUO, Yuxing, Hangzhou, Zhejiang 310052 (CN)
(74) Representative: Vogelbruch, Keang
(86) International application number: PCT/CN2024/097364
(87) International publication number: WO 2025/236339

(57) **Abstract**

The present invention relates to the field of medical instruments, and provides a digestive tract stent retrieval apparatus and a sleeve for an endoscope. The digestive tract stent retrieval apparatus comprises a detachable handle, a tube body, a trumpet-shaped protective sleeve, and an endoscope retrieval cap configured to be mounted on a distal end of an endoscope. Two ends of the tube body are a first connection end and a second connection end, respectively. The first connection end is connected to the detachable handle, and the second connection end is connected to the trumpet-shaped protective sleeve. The inner diameter of the second connection end is greater than the inner diameter of the tube body. The endoscope retrieval cap is trumpet-shaped. The endoscope retrieval cap extends out of the tube body and, under a pulling force, carries a digestive tract stent into the tube body. The present invention can reduce the risk of upper digestive tract injury during stent removal, improve treatment safety, and allow separation of the stent and the retrieval apparatus using the detachable handle after stent removal.

## Description

### CROSS-REFERENCE TO RELATED APPLICATIONS

This invention claims priority to Chinese Patent Application No. 2024106183086, filed with the China National Intellectual Property Administration on May 17, 2024, entitled" DIGESTIVE TRACT STENT RETRIEVAL APPARATUS AND SLEEVE FOR ENDOSCOPE", the entire contents of which are incorporated herein by reference.

### TECHNICAL FIELD

The present invention relates to the field of medical devices, and in particular, to a digestive tract stent retrieval device (i.e., a digestive tract stent retrieval apparatus) and an endoscope tube (i.e., a sleeve for endoscope).

### BACKGROUND ART

With the development of society and the improvement of living standards, obesity and diabetes have become increasingly serious global health issues. Currently, the treatment methods for obesity and diabetes mainly include medication, dietary control, and surgical operations. Among these, surgical operations are an effective treatment modality, but they carry significant surgical risks and cause irreversible damage to the human physiological structure as well as postoperative complications. Therefore, it is of great significance to explore a safe and effective non-surgical treatment method.

In recent years, the duodenal-jejunal bypass liner technology has achieved remarkable results in the treatment of obesity and diabetes. This technology fixes the proximal end of a tube via a stent and then places the tube between the duodenum and jejunum to restrict the digestion and absorption of food, thereby achieving the goals of weight loss and blood glucose reduction. Since the stent needs to be fixed at the upper end of the duodenum to secure the tube and prevent its dislodgement, the stent is often designed with an anchoring spike structure to prevent self-displacement for better fixation. However, this structure poses great challenges to stent retrieval.

In current retrieval methods, the stent is usually pulled out of the digestive tract by physical force. As stents used for fixing duodenal-jejunal bypass liners are often designed with anti-displacement structures such as spikes and cutting edges, such retrieval operations relying directly on physical force can lacerate the digestive tract wall during the retrieval process, leading to severe complications such as bleeding and perforation. At present, this technology has problems such as upper digestive tract injury during stent retrieval, which poses certain risks to patients. In addition, as products of this type are implantable devices, research on clinically retrieved specimens is often required to realize continuous improvement and design upgrading of the products. However, current retrieval methods involve directly discarding them as waste, which precludes separation and subsequent research.

In view of the above, the present invention is hereby proposed.

### SUMMARY

The objectives of the present invention include, for example, providing a digestive tract stent retrieval device and an endoscope tube, which can reduce the risk of upper digestive tract injury during stent retrieval, improve treatment safety, and facilitate the separation of the stent from the retrieval device after the stent is retrieved.

Embodiments of the present invention can be implemented as follows.

In a first aspect, the present invention provides a digestive tract stent retrieval device, comprising a detachable handle, a tube body, a trumpet-shaped protective sleeve, and an endoscope retrieval cap configured to be mounted at the distal end of an endoscope, wherein the tube body has a first connecting end and a second connecting end at its two ends respectively, the first connecting end is connected to the detachable handle, the second connecting end is connected to the trumpet-shaped protective sleeve, the inner diameter of the second connecting end is larger than that of the tube body, the endoscope retrieval cap is trumpet-shaped, and the endoscope retrieval cap extends out of the tube body and carries the digestive tract stent into the tube body under a pulling force.

Optionally, the inner cone angle of the endoscope retrieval cap is smaller than that of the trumpet-shaped protective sleeve.

Optionally, the inner cone angle of the endoscope retrieval cap is 25°-40°.

Optionally, the inner cone angle of the trumpet-shaped protective sleeve is 30°-50°.

Optionally, the length of the endoscope retrieval cap is 2 mm-10 mm smaller than that of the trumpet-shaped protective sleeve.

Optionally, the minimum diameter of the endoscope retrieval cap is 4 mm-8 mm smaller than that of the trumpet-shaped protective sleeve.

Optionally, the endoscope retrieval cap has a hardness of 30A -75A and a thickness of 0.4 mm-1.8 mm.

Optionally, the tube body has an inner diameter of 10 mm-19 mm and an outer diameter of 12 mm-21 mm, and after the second connecting end is connected to the tube body, the inner diameter of the second connecting end is 0.1 mm-4 mm larger than that of the tube body.

Optionally, the materials of the trumpet-shaped protective sleeve and the endoscope retrieval cap comprise one of silica gel and polyurethane.

Optionally, a first hydrophilic coating is provided on the inner wall of the trumpet-shaped protective sleeve and the outer wall of the endoscope retrieval cap, and the curing method of the first hydrophilic coating is photocuring.

Optionally, the photocuring duration is 1 min-15 min.

Optionally, the materials of the trumpet-shaped protective sleeve and the endoscope retrieval cap respectively comprise one of silica gel and polyurethane.

Optionally, a second hydrophilic coating is provided on the inner wall of the tube body, and the curing method of the second hydrophilic coating is thermal curing.

Optionally, for the thermal curing, the curing temperature is 50°C~70°C, and the curing duration is 10 min-120 min, preferably 40 min-80 min.

Optionally, the detachable handle comprises a handle body, a handle cover and a sealing ring, one end of the handle body is detachably connected to the handle cover, the other end thereof is fixedly connected to the tube body, and the sealing ring is arranged between the handle cover and the handle body.

Optionally, the retrieval force for pulling the digestive tract stent into the endoscope retrieval cap is not greater than 40N, preferably less than 30N.

In a second aspect, the present invention provides an endoscope tube, comprising the digestive tract stent retrieval device according to any one of the aforementioned embodiments.

The embodiments of the present invention include, for example, the following beneficial effects.

The present invention provides a digestive tract stent retrieval device, which uses the endoscope retrieval cap to protect the grasped digestive tract stent, enabling the grasped digestive tract stent to cross the pylorus under protection and avoiding injury to the pylorus during retrieval. Meanwhile, after reaching the stomach, under the lubricating effect provided by the hydrophilic coating, the special design of the second connecting end and the trumpet-shaped protective sleeve facilitates the entry of the endoscope retrieval cap carrying the digestive tract stent into the trumpet-shaped protective sleeve. The trumpet-shaped protective sleeve provides a second protection, greatly reducing the force required to pull the stent into the tube, with the retrieval force not exceeding 40 N, which can protect the cardiac orifice to the maximum extent (avoiding injury to the cardiac orifice) when pulling the stent into the tube body. Under the protection of the tube body, injury to the esophagus caused by the digestive tract stent during withdrawal from the body can be avoided. In addition, through the detachable structural design of the detachable handle itself, the embodiments of the present invention allow the detachable handle to be separated after the digestive tract stent is withdrawn from the body, facilitating the removal of the stent for subsequent research, and enabling continuous product upgrading and iteration to improve adverse reactions of subsequent products.

### BRIEF DESCRIPTION OF DRAWINGS

FIG. 1 is a schematic structural view of the digestive tract stent retrieval device provided by the present invention when the endoscope retrieval cap is not installed;
FIG. 2 is an exploded view of the digestive tract stent retrieval device provided by the present invention;
FIG. 3 is a schematic structural view of the endoscope retrieval cap of the digestive tract stent retrieval device provided by the present invention;
FIG. 4 is an assembly view of the digestive tract stent retrieval device provided by the present invention in an operating state;
FIG. 5 is a diagram of a human body model used in experiments with the digestive tract stent retrieval device provided by the present invention.

Reference numerals: 100-digestive tract stent retrieval device; 110-detachable handle; 111-handle body; 112-handle cover; 113-sealing ring; 120-tube body; 121-first connecting end; 122-second connecting end; 130-trumpet-shaped protective sleeve; 131-connecting column; 132-trumpet sleeve; 140-endoscope retrieval cap;
200-endoscope.

### DETAILED DESCRIPTION OF EMBODIMENTS

To clarify the objectives, technical solutions, and advantages of the embodiments of the present invention, the technical solutions in the embodiments of the present invention will be clearly and completely described below in conjunction with the accompanying drawings in the embodiments of the present invention. Obviously, the described embodiments are part rather than all of the embodiments of the present invention. Generally, the components of the embodiments of the present invention described and illustrated in the accompanying drawings herein may be arranged and designed in various different configurations.

Therefore, the following detailed description of the embodiments of the present invention provided in the accompanying drawings is not intended to limit the scope of the claimed present invention, but merely represents selected embodiments of the present invention. Based on the embodiments in the present invention, all other embodiments obtained by those of ordinary skill in the art without making creative efforts shall fall within the protection scope of the present invention.

It should be noted that similar reference numerals and letters indicate similar items in the following accompanying drawings. Therefore, once an item is defined in one accompanying drawing, it is not necessary to further define and explain it in subsequent accompanying drawings.

In the description of the present invention, it should be noted that if the terms "upper", "lower", "inner", "outer" and the like appear to indicate orientations or positional relationships, they are based on the orientations or positional relationships shown in the accompanying drawings, or the orientations or positional relationships commonly placed when the product of the present invention is used. They are only for the convenience of describing the present invention and simplifying the description, rather than indicating or implying that the referred device or element must have a specific orientation, be constructed and operated in a specific orientation, and thus should not be construed as limiting the present invention.

In addition, if the terms "first", "second" and the like appear, they are only configured to distinguish descriptions, and cannot be understood as indicating or implying relative importance.

It should be noted that where there is no conflict, the features in the embodiments of the present invention may be combined with each other.

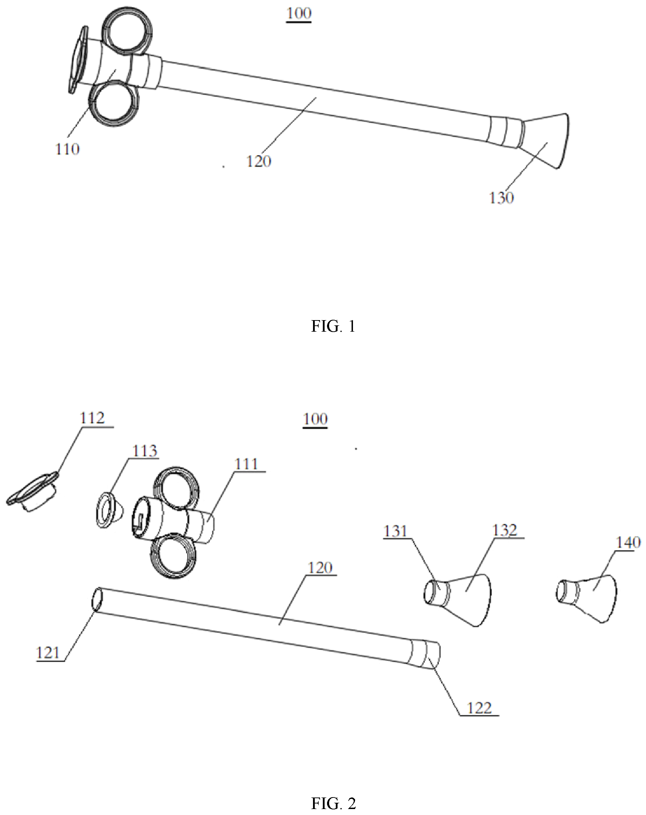

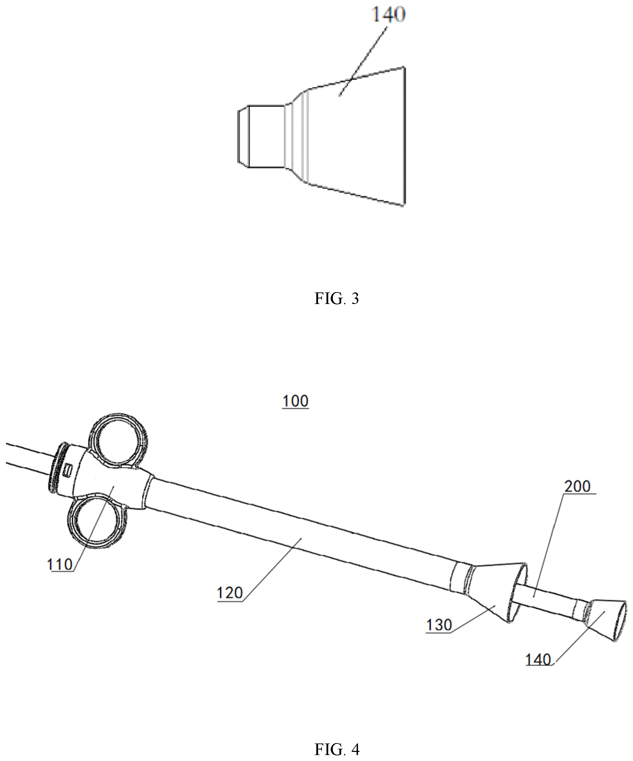

Please refer to FIGS. 1, 2, 3, and 4. The present invention provides a digestive tract stent retrieval device 100, which includes a detachable handle 110, a tube body 120, a trumpet-shaped protective sleeve 130, and an endoscope retrieval cap 140 configured to be mounted at the distal end of an endoscope.

In the present invention, the tube body 120 is a hollow tubular structure. The two ends of the tube body 120 are a first connecting end 121 and a second connecting end 122 respectively. The first connecting end 121 is connected to the detachable handle 110, and the second connecting end 122 is connected to the trumpet-shaped protective sleeve 130. The detachable arrangement of the detachable handle 110 itself enables the digestive tract stent retrieval device 100 to be easily disassembled after use, thereby facilitating the removal of the retrieved digestive tract stent inside for subsequent research.

Here, the inner diameter of the second connecting end 122 is larger than that of the tube body 120. The tube body 120 has an inner diameter of 10 mm-19 mm and an outer diameter of 12 mm-21 mm. After the second connecting end 122 is connected to the tube body 120, the inner diameter of the second connecting end 122 is 0.1 mm-4 mm larger than that of the tube body 120. In this embodiment, the enlarged inner diameter of the second connecting end 122 facilitates the insertion of the trumpet-shaped protective sleeve 130 into the second connecting end 122. Specifically, the trumpet-shaped protective sleeve 130 includes a connecting column 131 and a trumpet sleeve 132 that are connected to each other, and the connecting column 131 is bonded to the inner side of the second connecting end 122. Through the above design, there is no step between the end of the trumpet-shaped protective sleeve 130 (i.e., the position of the connecting column 131) and the tube body 120, avoiding the situation where the digestive tract stent gets stuck.

The endoscope retrieval cap 140 is trumpet-shaped. During the installation of the digestive tract stent retrieval device 100, the endoscope 200 is first inserted into the tube body 120 until it fully passes through the trumpet-shaped protective sleeve 130. Subsequently, the endoscopic retrieval cap 140 is mounted on the distal end of the endoscope 200. In operation, the endoscope grasps the digestive tract stent, which is then pulled into the endoscopic retrieval cap 140. The endoscopic retrieval cap 140 serves to enclose the digestive tract stent and prevent it from puncturing the digestive tract wall. After being enclosed within the endoscopic retrieval cap 140, the digestive tract stent is pulled out along the trumpet-shaped protective sleeve 130 toward the detachable handle 110.

In the present invention, the endoscope retrieval cap 140 protects the grasped digestive tract stent, enabling the grasped digestive tract stent to cross the pylorus under protection and avoiding injury to the pylorus during retrieval. Meanwhile, the opening of the trumpet-shaped protective sleeve 130 facilitates the entry of the endoscope retrieval cap 140 carrying the digestive tract stent, thereby facilitating pulling into the tube body 120.

In the present invention, the structures of the endoscope retrieval cap 140 and the trumpet-shaped protective sleeve 130 are strictly controlled. Specifically, the inner cone angle of the endoscope retrieval cap 140 is smaller than that of the trumpet-shaped protective sleeve 130; optionally, the inner cone angle of the endoscope retrieval cap 140 is 25°-40°, and the inner cone angle of the trumpet-shaped protective sleeve 130 is 30°-50°. By controlling the inner cone angles of the endoscope retrieval cap 140 and the trumpet-shaped protective sleeve 130, the opening of the trumpet-shaped protective sleeve 130 is larger than that of the endoscope retrieval cap 140, facilitating the entry of the endoscope retrieval cap 140 into the trumpet-shaped protective sleeve 130.

In addition, in the present invention, the length of the endoscope retrieval cap 140 is 2 mm-10 mm shorter than that of the trumpet-shaped protective sleeve 130, and the minimum diameter of the endoscope retrieval cap 140 is 4 mm-8 mm smaller than that of the trumpet-shaped protective sleeve 130. By controlling the lengths and minimum diameters of the endoscope retrieval cap 140 and the trumpet-shaped protective sleeve 130, the endoscope retrieval cap 140 can be completely enclosed within the trumpet-shaped protective sleeve 130, facilitating the entry of the endoscope retrieval cap 140 into the trumpet-shaped protective sleeve 130. Further, in the present invention, endoscope retrieval cap 140 has a hardness of 30A-75A and a thickness of 0.4 mm-1.8 mm. By selecting a material with moderate hardness and proper thickness to prepare the endoscope retrieval cap 140, it has a certain elasticity while maintaining a certain hardness, facilitating the encasement of the digestive tract stent to be retrieved.

Further, in the present invention, hydrophilic coatings are provided on the inner wall of the tube body 120, the inner wall of the trumpet-shaped protective sleeve 130, and the outer wall of the endoscope retrieval cap 140. The hydrophilic coatings include but are not limited to PVP coatings. Optionally, different curing methods may be selected for the hydrophilic coatings according to the different surface materials to which they are applied, such as thermal curing or photocuring. Herein, the temperature for thermal curing is 50°C~70°C, and the curing duration is 10 min-120 min, preferably 40 min-80 min. The duration for photocuring is 1 min-15 min.

Specifically, the material of the tube body 120 includes but is not limited to one or more of polyvinyl chloride, silica gel, polyurethane, nylon, and polyethylene; the curing method of the hydrophilic coating on the tube body 120 is thermal curing. The materials of the trumpet-shaped protective sleeve 130 and the endoscope retrieval cap 140 respectively include but are not limited to one of silica gel and polyurethane; the curing method of the hydrophilic coatings on the trumpet-shaped protective sleeve 130 and the endoscope retrieval cap 140 is photocuring.

Through the protection from the aforementioned dual structures and the provision of the hydrophilic coatings, the present invention can achieve a retrieval force not more than 40 N for pulling the digestive tract stent into the endoscope retrieval cap 140; preferably less than 30 N. This greatly reduces the force required to pull the stent into the tube, and can protect the cardiac orifice to the maximum extent (avoiding injury to the cardiac orifice).

In addition, the detachable handle 110 in the present invention includes a handle body 111, a handle cover 112, and a sealing ring 113. One end of the handle body 111 is detachably connected to the handle cover 112, and the other end is fixedly connected to the tube body 120. The sealing ring 113 is disposed between the handle cover 112 and the handle body 111. After the digestive tract stent is withdrawn from the body, the detachable structural design between the handle body 111 and the handle cover 112 allows the detachable handle 110 to be separated, facilitating the removal of the stent for subsequent research, and enabling continuous product upgrading and iteration to reduce adverse reactions of subsequent products.

It should be understood that the detachable structural design in the present invention is not limited to the detachability of the detachable handle 110 itself. Any design that enables the retrieved digestive tract stent to be removed from the digestive tract stent retrieval device 100 is acceptable. For example, the connection between the detachable handle 110 and the tube body 120 may also be designed to be detachable.

In addition, the present invention also provides an endoscope tube, which includes the aforementioned digestive tract stent retrieval device 100.

The technical solution of the present invention is further elaborated below in conjunction with specific examples.

### Example 1

This example provides a digestive tract stent retrieval device 100, which includes a detachable handle 110, a tube body 120, a trumpet-shaped protective sleeve 130, and an endoscope retrieval cap 140 configured to be mounted at the distal end of an endoscope 200.

Here, the detachable handle 110 includes a handle body 111, a handle cover 112, and a sealing ring 113. One end of the handle body 111 is detachably connected to the handle cover 112, and the other end is fixedly connected to the tube body 120. The sealing ring 113 is disposed between the handle cover 112 and the handle body 111.

The tube body 120 has an inner diameter of 14 mm and an outer diameter of 16 mm. The outer diameter of the second connecting end 122 is 16 mm, and the inner diameter of the second connecting end 122 after being connected to the trumpet-shaped protective sleeve 130 is 14 mm.

The trumpet-shaped protective sleeve 130 is trumpet-shaped with a length of 38 mm. The inner diameter at the minimum diameter of the trumpet-shaped protective sleeve 130 is 14 mm, and the inner cone angle of the trumpet-shaped protective sleeve 130 is 37°.

The endoscope retrieval cap 140 is trumpet-shaped with a length of 28 mm. The outer diameter at the minimum outer diameter of the endoscope retrieval cap 140 is 12 mm, and the inner cone angle of the endoscope retrieval cap 140 is 32°. The silica gel hardness of the endoscope retrieval cap 140 is 35A, and the wall thickness is 1.2 mm.

The material of the tube body 120 is polyvinyl chloride. The materials of the sealing ring 113, the trumpet-shaped protective sleeve 130, and the endoscope retrieval cap 140 are silica gel. Hydrophilic coatings (PVP coatings) are provided on the inner wall of the tube body 120, the inner wall of the trumpet-shaped protective sleeve 130, and the outer wall of the endoscope retrieval cap 140. The curing method of the hydrophilic coating inside the tube body 120 is thermal curing at a temperature of 60°C for a curing duration of 50 min. The curing method for the endoscope retrieval cap 140 and the trumpet-shaped protective sleeve 130 is photocuring with a photocuring duration of 5 min.

### Example 2

This example is basically the same as Example 1, with the only difference being that in this example, the wall thickness of the endoscope retrieval cap 140 is 0.4 mm.

### Example 3

This example is basically the same as Example 1, with the only difference being that in this example, the silica gel hardness of the endoscope retrieval cap 140 is 60A, and the wall thickness is 1 mm.

### Example 4

This example is basically the same as Example 1, with the only difference being that in this example, the trumpet-shaped protective sleeve 130 is trumpet-shaped with a length of 38 mm, the inner diameter at the minimum diameter of the trumpet-shaped protective sleeve 130 is 14 mm, and the inner cone angle of the trumpet-shaped protective sleeve 130 is 40°; the endoscope retrieval cap 140 is trumpet-shaped with a length of 32 mm, the outer diameter at the minimum outer diameter of the endoscope retrieval cap 140 is 12 mm, and the inner cone angle of the endoscope retrieval cap 140 is 35°.

### Example 5

This example is basically the same as Example 1, with the only difference being that in this example, the trumpet-shaped protective sleeve 130 is trumpet-shaped with a length of 38 mm, the inner diameter at the minimum diameter of the trumpet-shaped protective sleeve 130 is 14 mm, and the inner cone angle of the trumpet-shaped protective sleeve 130 is 40°; the endoscope retrieval cap 140 is trumpet-shaped with a length of 35 mm, the outer diameter at the minimum outer diameter of the endoscope retrieval cap 140 is 10 mm, and the inner cone angle of the endoscope retrieval cap 140 is 35°.

### Comparative Example 1

This comparative example is basically the same as Example 1, with the only difference being that in this comparative example, the inner diameter of the second connecting end 122 after being connected to the trumpet-shaped protective sleeve 130 is consistent with the inner diameter of the tube body 120. Specifically, in this comparative example, the inner diameter of the tube body 120 is 12 mm and the outer diameter is 14 mm; the diameter of the second connecting end 122 is 16 mm, and the inner diameter of the second connecting end 122 after being connected to the trumpet-shaped protective sleeve 130 is 12 mm.

### Comparative Example 2

This comparative example is basically the same as Example 1, with the only difference being that in this comparative example, no hydrophilic coating is provided on the inner wall of the tube body 120, the inner wall of the trumpet-shaped protective sleeve 130, or the outer wall of the endoscope retrieval cap 140.

### Comparative Example 3

This comparative example is basically the same as Example 1, with the only difference being that in this comparative example, no trumpet-shaped protective sleeve is provided.

### Comparative Example 4

This comparative example is basically the same as Example 1, with the only difference being that in this comparative example, no endoscope retrieval cap is provided.

### Experimental Example

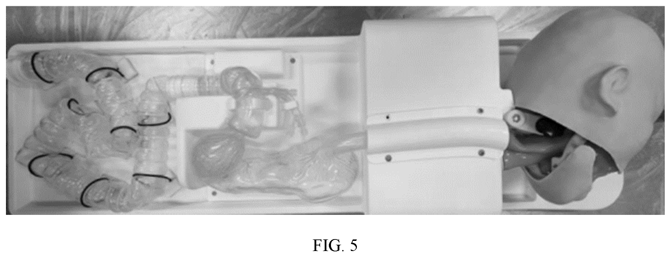

The digestive tract stent retrieval devices 100 of Examples 1-5 and Comparative Examples 1-4 were used. First, the endoscope 200 was inserted into the tube body 120 until it completely passed through the trumpet-shaped protective sleeve 130, and then the endoscope retrieval cap 140 was mounted at the distal end of the endoscope 200 (no endoscope retrieval cap 140 was provided in Comparative Example 4, so there was no need to mount the endoscope retrieval cap 140). Using a human body model as shown in FIG. 5, the device was inserted into the stomach through the oral cavity of the human body model. Then, the tube body 120 and the detachable handle 110 reserved outside the body were fixed, and the endoscope was advanced to the pylorus. An endoscope retrieval grasper was inserted through the instrument channel of the endoscope itself to shrink the digestive tract stent to be retrieved and pull it into the protection range of the endoscope retrieval cap 140, and then withdraw it together with the endoscope to the vicinity of the trumpet-shaped protective sleeve 130 in the stomach. The digestive tract stent to be retrieved was then pulled into the tube body 120, and the maximum force F required to pull it into the tube body 120 was recorded. The test was repeated 15 times to calculate the success rate for each time, and scratches on the esophagus/intestinal tract were observed simultaneously.

| Example | F/N | Success Rate/% | Were There Scratches on the Esophagus/Intestine? |
|---|---|---|---|
| Example 1 | 13.5 | 100% | No |
| Example 2 | 2.9 | 100% | No |
| Example 3 | 18.6 | 100% | No |
| Example 4 | 16.8 | 100% | No |
| Example 5 | 13.7 | 100% | No |
| Comparative Example 1 | 45.8 | 100% | No |
| Comparative Example 2 | >50 | 100% | No |
| Comparative Example 3 | 25.3 | 60% | No |
| Comparative Example 4 | 17.3 | 100% | Yes |

As can be seen from the above table, during the simulated use, Examples 1-5 all provide excellent protection for the esophagus and intestinal tract, and the retrieval force is less than 20 N. When the diameter of the second connecting end after connection is the same as the diameter of the tube body (Comparative Example 1), the retrieval force increases significantly due to the lack of effective guidance. When no hydrophilic coating is provided, the surface friction increases, leading to a significant increase in retrieval force. Meanwhile, when no trumpet-shaped protective sleeve is provided, although the retrieval force is not large, the retrieval cap carrying the stent cannot be pulled into the retrieval tube in one go due to the lack of guidance from the trumpet-shaped protective sleeve. In addition, when no retrieval cap is provided, certain damage to the intestinal tract is caused.

In summary, the embodiments of the present invention provide a digestive tract stent retrieval device 100, which uses the endoscope retrieval cap 140 to protect the grasped digestive tract stent, enabling the grasped digestive tract stent to cross the pylorus under protection and avoiding injury to the pylorus during retrieval. Meanwhile, after reaching the stomach, under the lubricating effect provided by the hydrophilic coating, the special design of the second connecting end 122 and the trumpet-shaped protective sleeve 130 facilitates the entry of the endoscope retrieval cap 140 carrying the digestive tract stent into the trumpet-shaped protective sleeve 130. The trumpet-shaped protective sleeve 130 provides a second protection, greatly reducing the force required to pull the stent into the tube, with the retrieval force not exceeding 40 N, which can protect the cardiac orifice to the maximum extent (avoiding injury to the cardiac orifice) when pulling the stent into the tube body 120. Under the protection of the tube body 120, injury to the esophagus caused by the digestive tract stent during withdrawal from the body can be avoided. In addition, through the detachable structural design of the detachable handle 110 itself, the embodiments of the present invention allow the detachable handle 110 to be separated after the digestive tract stent is withdrawn from the body, facilitating the removal of the stent for subsequent research, and enabling continuous product upgrading and iteration to improve adverse reactions of subsequent products.

The above description is merely specific embodiments of the present invention, but the protection scope of the present invention is not limited thereto. Any changes or substitutions easily conceivable by those skilled in the art within the technical scope disclosed by the present invention shall be covered within the protection scope of the present invention. Therefore, the protection scope of the present invention shall be subject to the protection scope of the claims.

### INDUSTRIAL PRACTICALITY

The digestive tract stent retrieval device provided by the present invention uses the endoscope retrieval cap to protect the grasped digestive tract stent, enabling the grasped digestive tract stent to cross the pylorus under protection and avoiding injury to the pylorus during retrieval. Meanwhile, after reaching the stomach, under the lubricating effect provided by the hydrophilic coating, the special design of the second connecting end and the trumpet-shaped protective sleeve facilitates the entry of the endoscope retrieval cap carrying the digestive tract stent into the trumpet-shaped protective sleeve. The trumpet-shaped protective sleeve provides a second protection, greatly reducing the force required to pull the stent into the tube, with the retrieval force not exceeding 40 N, which can protect the cardiac orifice to the maximum extent (avoiding injury to the cardiac orifice) when pulling the stent into the tube body. Under the protection of the tube body, injury to the esophagus caused by the digestive tract stent during withdrawal from the body can be avoided.

## Claims

1. A digestive tract stent retrieval device, **characterized by** comprising a detachable handle, a tube body, a trumpet-shaped protective sleeve, and an endoscope retrieval cap configured to be mounted at a distal end of an endoscope, wherein the tube body has a first connecting end and a second connecting end at two ends respectively, the first connecting end is connected to the detachable handle, the second connecting end is connected to the trumpet-shaped protective sleeve, an inner diameter of the second connecting end is larger than that of the tube body, the endoscope retrieval cap is trumpet-shaped, and the endoscope retrieval cap extends out of the tube body and carries the digestive tract stent into the tube body under a pulling force.

2. The digestive tract stent retrieval device according to claim 1, wherein an inner cone angle of the endoscope retrieval cap is smaller than that of the trumpet-shaped protective sleeve;
optionally, the inner cone angle of the endoscope retrieval cap is 25°-40°;
optionally, the inner cone angle of the trumpet-shaped protective sleeve is 30°-50°.

3. The digestive tract stent retrieval device according to claim 1, wherein a length of the endoscope retrieval cap is 2 mm-10 mm smaller than that of the trumpet-shaped protective sleeve;
optionally, a minimum diameter of the endoscope retrieval cap is 4 mm-8 mm smaller than that of the trumpet-shaped protective sleeve.

4. The digestive tract stent retrieval device according to claim 1, wherein the endoscope retrieval cap has a hardness of 30A-75A and a thickness of 0.4 mm-1.8 mm.

5. The digestive tract stent retrieval device according to claim 1, wherein the tube body has an inner diameter of 10 mm-19 mm and an outer diameter of 12 mm-21 mm, and after the second connecting end is connected to the tube body, an inner diameter of the second connecting end is 0.1 mm-4 mm larger than that of the tube body.

6. The digestive tract stent retrieval device according to claim 1, wherein a material of the tube body comprises one or more of polyvinyl chloride, silica gel, polyurethane, nylon and polyethylene;
optionally, a first hydrophilic coating is provided on an inner wall of the trumpet-shaped protective sleeve and an outer wall of the endoscope retrieval cap, and a curing method of the first hydrophilic coating is photocuring;
optionally, the photocuring duration is 1 min-15 min.

7. The digestive tract stent retrieval device according to claim 6, wherein materials of the trumpet-shaped protective sleeve and the endoscope retrieval cap respectively comprise one of silica gel and polyurethane;
optionally, a second hydrophilic coating is provided on an inner wall of the tube body, and a curing method of the second hydrophilic coating is thermal curing;
optionally, for the thermal curing, a curing temperature is 50°C~70°C, and a curing duration is 10 min-120 min, preferably 40 min-80 min.

8. The digestive tract stent retrieval device according to claim 1, wherein the detachable handle comprises a handle body, a handle cover and a sealing ring, one end of the handle body is detachably connected to the handle cover, the other end of the handle body is fixedly connected to the tube body, and the sealing ring is arranged between the handle cover and the handle body.

9. The digestive tract stent retrieval device according to claim 1, wherein a retrieval force for pulling the digestive tract stent into the endoscope retrieval cap is not greater than 40N, preferably less than 30N.

10. An endoscope tube, **characterized by** comprising the digestive tract stent retrieval device according to any one of claims 1-9.
